# EUROPÄISCHE PATENTANMELDUNG

(11) **EP 2 617 348 A1**
(43) Veröffentlichungstag der Anmeldung: **24.07.2013**
(21) Anmeldenummer: 13000222.3
(22) Anmeldetag: 16.01.2013
(51) Int. Cl.: A61B 1/00, A61B 1/07, G02B 23/24, G02B 6/06

(54) **Lichtleiter mit einem Bündel von lichtleitenden Fasern und ein Verfahren zur Biegung des Lichtleiters**

(30) Priorität: 20.01.2012 DE 102012200794
(71) Anmelder: Karl Storz GmbH & Co. KG, 78532 Tuttlingen (DE)
(72) Erfinder: Dahmen, Jan, 78532 Tuttlingen (DE)

(57) **Zusammenfassung**

Die Erfindung betrifft einen Lichtleiter (1) mit einem Bündel (2) von lichtleitenden Fasern (2) zum Übertragen von Licht und ein Verfahren zum Biegen des aus lichtleitenden Fasern gebildeten Lichtleiters. Der Lichtleiter (1) mit einem Bündel (2) von lichtleitenden Fasern (6) zum Übertragen von Licht einer an einem ersten Endabschnitt (13) des Lichtleiters (1) ankoppelbaren Lichtquelle zu einem zweiten Endabschnitt (12) des Lichtleiters (1), weist einen ersten Abschnitt (3) und einen als Verbundabschnitt (5) ausgebildeten gekrümmten zweiten Abschnitt (4) auf, wobei die lichtleitenden Fasern (6) in dem zweiten Abschnitt (4) mittels eines ausgehärteten Klebemittels (8) bei Aufrechterhaltung der Krümmung spannungsfrei gegeneinander fixiert sind.

## Beschreibung

Die Erfindung betrifft einen Lichtleiter mit einem Bündel von lichtleitenden Fasern zum Übertragen von Licht und ein Verfahren zum Biegen des aus lichtleitenden Fasern gebildeten Lichtleiters.

Lichtleiter aus Faserbündeln zur Übertragung von Licht können zur Signal oder Lichtübertragung verwendet werden.

DE202008001786 beschreibt eine Aufzugsanlage, bei der in den Zugseilen zur Positionsfindung der Aufzugskabine oder der Überwachung eines Tragriemens Glasfaserleiter verwendet werden.

Es kann vorkommen, dass die Faserbündel dauerhaft gebogen werden müssen, so dass der Lichtleiter das Licht bzw. Signal auch an die gewünschte Stelle übertragen kann.

Aus dem allgemeinen Stand der Technik sind Verfahren zum Biegen von Faserbündeln zum Lichtleiten bekannt. Beispielsweise wird nach dem Stand der Technik das Biegen oder Verformen des Faserbündels mit Hilfe eines Biegekeils durchgeführt. Üblicherweise verbleibt der Biegekeil nach dem Biegevorgang in dem gebogenen Faserbündels und wird mit demselben verklebt. Es besteht jedoch die Gefahr, dass der Biegekeil nicht ausreichend von dem ausgehärteten Klebstoff festgehalten wird und aus dem Verbund aus Klebemittel und Glasfasern herausfallen kann. Dies kann insbesondere dann nachteilig sein, wenn die lichtleitenden Fasern in einem medizinischen Gerät verwendet werden. Der losgelöste Keil kann im Extremfall in den Patienten fallen.

Wenn der Biegekeil nach dem Biegevorgang aus dem gebogenen Faserbündel beseitigt wird, entsteht eine Fehlstelle, die mit Klebstoff ausgefüllt werden muss. Das zusätzliche Einbringen von Klebstoff in die Fehlstelle kann zu einem schlechten Verbund zwischen dem Klebstoff und dem Faserbündel führen. Dies kann zu unterschiedlichen Festigkeiten des Faserbündels führen, insbesondre dann, wenn der Klebstoff zu Stellen in dem Faserbündel wandert, die die Längsausdehnung des Faserbündels bei Druck- und Temperaturschwankungen einschränken und die mechanische Belastbarkeit herabsetzen, wie diese beispielsweise beim Sterilisieren durch einen Autoklaven vorkommen kann. Durch die mangelnde Möglichkeit einer Längsausdehnung kann es zum Bruch einzelner Fasern des Faserbündels kommen.

Ein anderes Verfahren zum Biegen von Faserbündeln von Lichtleiten sieht vor, dass das Biegen bzw. Verformen des Faserbündels unter Einbeziehung der Geometrie des zur Aufnahme des Faserbündels vorgesehen Vorrichtungs-Gehäuseabschnittes erfolgt. Das Biegen der Faser erfolgt in diesem Fall mit Hilfe entsprechender Führungs- und Aufnahmeabschnitte in dem Vorrichtungsgehäuse. Dies erfordert eine aufwendige Gehäusegeometrie, die aufwendig und kostenintensiv in der Planung und Herstellung sein kann. Üblicherweise werden bei diesem Verfahren die Glasfaserbündel in der Gehäusegeometrie mittels eines Klebstoffes fixiert, wobei auch hier nicht sichergestellt werden kann, ob der Klebstoff an der hierfür vorgesehenen Position bleibt und unerwünschterweise durch die Kapillarwirkung des Faserbündels zu anderen Stellen abwandert. Dies kann wiederum dazu führen, dass fixierende Zwangsstellen in dem Faserbündel erzeugt werden, die insbesondere beim Autoklavieren nachteilige Zug-, Druck und Querspannungen im dem Faserbündel erzeugen können.

Somit ist es Aufgabe der vorliegenden Erfindung, einen Lichtleiter bzw. ein Verfahren bereitzustellen, mit dem die oben genannten Nachteile gelöst werden können.

Diese Aufgabe wird mit den Merkmalen der unabhängigen Ansprüche gelöst. Weitere Ausführungsbeispiele sind in den aus auf diese rückbezogenen Unteransprüche angegeben.

Ein Gedanke der Erfindung ist, ein Lichtleiterbündel mit einem Bündel von lichtleitenden Fasern bereitzustellen, das einen ersten und einen zweiten Abschnitt aufweist, wobei der zweite Abschnitt eine vorbestimmte Biegung aufweist, die mittels eines Klebemittels aufrecht erhalten wird, wobei die einzelnen lichtleitenden Fasern im Wesentlichen spannungsfrei gegeneinander fixiert sind. Dies wird dadurch erreicht, dass zuerst ein pastöses Klebemittel in das Faserbündel eingebracht wird und danach der mit dem pastösen Klebemittel versehene Bereich des Klebemittels in die erforderliche Biegungsgestalt gebogen wird. Das noch unausgehärtete Klebemittel erlaubt, dass sich die einzelnen lichtleitenden Fasern beim Biegen gegeneinander verschieben können, so dass keine Zwangsspannungen in das Faserbündel eingebracht werden.

Der erfindungsgemäße Lichtleiter weist ein Bündel von lichtleitenden Fasern zum Übertragen von Licht von einer an einem ersten Endabschnitt des Lichtleiters ankoppelbaren Lichtquelle zu einem zweiten Endabschnitt des Lichtleiters auf, wobei der Lichtleiter einen ersten Abschnitt und einen als Verbindungsabschnitt ausgebildeten gekrümmten zweiten Abschnitt aufweist, wobei erfindungsgemäß die lichtleitenden Fasern in dem zweiten Abschnitt mittels eines ausgehärteten Klebemittels bei Aufrechterhaltung der Krümmung im Wesentlich spannungsfrei gegeneinander fixiert sind. D.h. die Fasern werden im Bereich des Verbundteils stabil gehalten, das weitgehend oder ganz spannungsfrei ist und einfach in einem Gehäuse eingebaut werden kann. In dem Gehäuse sind keine Halte-oder Stützvorrichtungen für den an sich stabilen Verbundabschnitt erforderlich. Dies hat den Vorteil, dass keine Zwangsspannungen in dem gekrümmten zweiten Abschnitt wegen der Biegung vorhanden sind, wodurch der Anteil an Faser, die beim Krümmen ansonsten brechen würden stark reduziert werden kann. Ein weiterer Vorteil besteht darin, dass der erfindungsgemäße Lichtleiter vor dem Einbau unaufwendig auf gebrochene Fasern kontrolliert werden kann, die kein Licht mehr weiterleiten können. Der Krümmungsverlauf des Verbundabschnittes kann aber auch geradlinig sein. Die Krümmung kann mit Hilfe eines entsprechenden Biegeradius r in der jeweiligen von dem X-Y-Z-Koordinatensystem aufgespannten Ebene angegeben werden, wobei der Biegeradius eine konstante oder eine unterschiedliche Krümmung aufweisen kann. Zum besseren Verständnis des Begriffes "Krümmung" wird auf nachfolgende Beispiele geometrischer Elemente mit unterschiedlicher Biegung hingewiesen: Die Krümmung einer Geraden ist überall gleich null, weil sich ihre Richtung nicht ändert. Ein Kreis mit dem Radius r hat überall eine gleiche Krümmung (nämlich 1/r), denn seine Richtung ändert sich überall gleich stark. Bei Kurven wechselt die Krümmung und entsprechend der Biegeradius von Kurvenpunkt zu Kurvenpunkt. Unter Krümmung ist in diesem Zusammenhang also eine Richtungsänderung pro durchlaufene Länge eines genügend kurzen Kurvenstücks der jeweiligen lichtleitenden Faser bzw. des Faserbündels zu verstehen.

Bei dem erfmdungsgemäßen Lichtleiters ist das Klebemittel derart in dem zweiten Abschnitt von lichtleitenden Fasern eingebracht, dass das Klebemittel die Außenumfangsflächen der lichtleitenden Fasern teilweise oder vollständig umgibt und miteinander verbindet, so dass das Klebemittel ein selbstragendes Stützgerüst bildet, in das einzelne oder mehrere oder alle lichtleitenden Fasern eingebettet sind, um den Verbundabschnitt zu bilden, so dass keine zusätzliche Stützvorrichtungen, insbesondere in einem Gehäuse, für den Verbundabschnitt erforderlich sind. Dies hat den Vorteil, dass die lichtleitenden Fasern von dem Stützgerüst aus ausgehärtetem Klebemittel geschützt und in der erforderlichen Biegung stabil gehalten werden, ohne dass in einem Gehäuse, in das der Lichtleiter eingebaut werden kann, zusätzliche Stützvorrichtungen vorhanden sein müssen. Somit kann ein einfacheres und unaufwändiger herzustellendes Gehäuse für den erfmdungsgemäßen Lichtleiter verwendet werden.

In einem weiteren erfindungsgemäßen Ausführungsbeispiel des Lichtleiters ist auf den zweiten Abschnitt ein flexibler Schlauch zur Stabilisierung desselben aufgebracht. Bei dem flexiblen Schlauch kann es sich um eine Ummantelung handeln, die eine stabilisierende Wirkung auf die am Außenumfang des Lichtleiters befmdenden lichtleitenden Fasern ausübt und gleichzeitig auch einen Schutz bewirkt, so dass das in dem Verbundabschnitt eingebrachte Klebemittel vor Substanzen geschützt ist, die ansonsten das Klebemittel angreifen würden. Insbesondere sollte die Ummantelung autoklavier-resistent sein und einen entsprechenden Schutz für den in dem Verbundabschnitt befindlichen Klebstoff bewirken können. Der flexible Schlauch hat vorrangig die Funktion, das Bündel aus lichtleitenden Fasern vor dem Aushärten des Klebemittels zu Stabilisieren und die im Wesentlichen kreisförmige Gestalt des Lichtleiterbündels zu halten und insbesondere die an der Außenumfangsfläche des Bündels liegenden lichtleitenden Fasern abzustützen, so dass diese in dem Verbundabschnitt verbleiben. Dies hat den Vorteil, dass die äußeren lichtleitenden Fasern sich nicht aus dem Verbundabschnitt lösen und insbesondere nicht brechen können. Ein weiterer Vorteil der Ummantelung ist, dass auf den Verbundabschnitt wirkende Belastungen nicht auf einzelne Fasern, sondern auf das gesamte Bündel verteilt werden. Der flexible Schlauch kann jede Ummantelung sein, die zum Stabilisieren der kreisförmigen Gestalt des Lichtleiterbündels geeignet ist, bevor das Klebemittel ausgehärtet ist. In einer bevorzugten Ausführungsform ist der flexible Schlauch ein Schrumpfschlauch. Ferner hat die Ummantelung die Funktion, die Handhabung des mit dem noch unausgehärteten Klebemittel versehen Verbundabschnittes zu erleichtern, insbesondere zu Verhindern, dass eine Biegevorrichtung mit dem Klebemittel beim Biegevorgang verschmutzt wird.

In einer weiteren bevorzugten Ausführungsform des erfindungsgemäßen Lichtleiters ist an dem ersten oder dem zweiten Endabschnitt des Lichtleiters eine Fassung zum Einsetzen in einer Aufnahme einer Vorrichtung oder eines Gehäuses einer Vorrichtung zur mechanischen Befestigung des Lichtleiters angebracht. Bei der Fassung handelt es sich bevorzugt um eine aufschiebbare Fassung, deren AußenDurchmesser mit dem Innen-Durchmesser einer Aufnahme beispielsweise in einem Beleuchtungsstab, einem Endoskop oder Exoskop korrespondiert. Wesentlich ist hierbei auch, dass die Fassung derart ausgebildet ist, dass die Fassung zur Verwendung in dem erfindungsgemäßen Verfahren zur Biegung des Lichtleiters, insbesondere in einer für das erfindungsgemäße Verfahren verwendeten Biegevorrichtung, geeignet ist.

In einer weiteren Ausführungsform des erfindungsgemäßen Lichtleiters ist die Fassung hülsenförmig ausgebildet und die Endungen der lichtleitenden Fasern an der Austrittsseite der Fassung weisen insgesamt eine konvexe und/oder konkave und/oder ebene Fläche auf, die vorzugsweise poliert oder geglättet ist. Dies hat den Vorteil, dass mit der Fassung beim Polieren keine einzelne Fasern aus dem Verbundabschnitt, insbesondere am Randbereich, unkontrollierbar wegbrechen können, was zu einer ungewünschten Lichtstreuung führen kann. Außerdem wird mit der Fassung an der Endung des Lichtleiters eine Steckerverbindung ausgebildet, die ein einfacheres Einsetzen der Endungen in korrespondierende Aufnahmen von Vorrichtungen erleichtert, in denen der erfindungsgemäße Lichtleiter verwendet wird.

In einer anderen Ausführungsform des erfindungsgemäßen Lichtleiters ist die Fassung aus einem Metall, Kunststoff oder einer Kombination aus Metall oder Kunststoff. Vorzugsweise ist die Fassung eine Hülse. Ferner soll das Metall, der Kunststoff oder die Kombination aus Metall oder Kunststoff für eine medizinische Anwendung geeignet und bio-kompatibel sein. Als Kunststoff kann insbesondere PEEK-Kunststoff verwendet werden. Wesentlich bei den verwendeten Materialien des erfmdungsgemäßen Lichtleiters ist, dass diese in einem Autoklaven medizinisch sterilisierbar sind und dass diese gegebenenfalls biokompatibel sind. Biokompatibel Materialien sind Materialien, die keinen negativen Einfluss und eine möglichst hohe Verträglichkeit gegenüber Patienten aufweisen. Der Schrumpfschlauch hat insbesondere die Funktion die Handhabung des mit dem unausgehärteten Klebemittel versehen Verbundabschnitt zu erleichtern und ein Verschmutzen mit Klebemittel von einer Biegevorrichtung zum Krümmen des Verbundabschnittes zu verhindern.

Nach einer anderen, bevorzugten Ausführungsform des erfmdungsgemäßen Lichtleiters liegt der aufgeschrumpfte Schlauch des Verbundabschnitts an der Fassung an. Dies hat insbesondere den Vorteil, dass zwischen Fassung und aufgeschrumpftem Schlauch keine Lücke entstehen kann und daher an dieser Stelle auch keine das Klebemittel angreifenden Substanzen eindringen können. Mit dem Begriff "anliegen" sind sowohl die Begriffe "benachbart" als auch "angrenzend" gemeint. Ferner kann der Schrumpfschlauch auch in die Fassung hineinragen und von dieser mehr oder weniger überlappt werden. Es ist auch vorstellbar, dass der Schrumpfschlauch direkt an den Außenrand der Fassung angrenzt oder mit einem geringen Abstand zu derselben beabstandet ist. Ferner hat der Schrumpfschlauch die Funktion, die Handhabung des mit dem noch unausgehärteten Klebemittel versehen Verbundabschnittes zu erleichtern, insbesondere zu Verhindern, dass eine Biegevorrichtung mit dem Klebemittel beim Biegevorgang verschmutzt wird.

In einer weiteren bevorzugten Ausführungsform des erfindungsgemäßen Lichtleiters sind der erste und der zweite Abschnitt derart ausgebildet, dass die lichtleitenden Fasern in dem ersten Abschnitt einen ersten Flächenquerschnitt aufweisen und in dem zweiten Abschnitt ein erstes zweites und oder weiteres Nebenbündel bilden, in die sich die lichtleitenden Fasern des ersten Abschnitts Y-förmig verzweigen und jeweils einen Flächenquerschnitt aufweisen, der kleiner als der erste Flächenquerschnitt ist. Es ist vorstellbar, dass die lichtleitenden Fasern in unterschiedlich große Flächenquerschnitte aufteilen, beispielsweise um einen dreizackförmigen Verbundabschnitt auszubilden. Es liegt im erfindungsgemäßen Gedanken, dass der Flächenquerschnitt des Hauptstranges in mehrere einzelne Nebenstränge aufgeteilt werden kann, der dann als Verbundabschnitt ausgebildet wird. Es ist jedoch auch im Bereich der Erfindung, dass der Hauptstrang unverzweigt bleibt und nur im Verbundabschnitt eine entsprechende Krümmung mit einem Biegeradius mit konstanter oder unterschiedlicher Krümmung aufweist.

Nach einem anderen erfindungsgemäßen Ausführungsbeispiel des Lichtleiters sind die lichtleitenden Fasern aus Glasfasern und/oder Kunststofffasern ausgebildet. Bevorzugterweise werden Glasfasern mit einem Glaskern verwendet, der einen Durchmesser etwa von 70 µm und eine Glas-Ummantelung mit etwa 2, 5µm aufweist. Die Dicke des Glasfaserkerns kann im Bereich von 20-500 µm aufweisen kann, wobei die Glasummantelung im Bereich von 3 bis 5 µm liegen kann. Lichtleitende Fasern aus Glas weisen den Vorteil auf, dass sie eine sehr hohe Lichttransmission mit einer sehr hohen Lichtausbeute aufweisen und gleichzeitig autoklavierbar sind. Es ist auch vorstellbar, dass der Glasfaserkern von einem Kunststoffmantel umgeben ist. Vorzugsweise weist der Kunststoff- oder Glasfasermantel einen Brechungsindex auf, der dem Brechungsindex von Luft nahekommt. Wenn, die Glasfasern, derart in dem erfindungsgemäßen Lichtleiter angeordnet sind, dass jede einzelne Faser an ihrer Außenumfangsfläche mit Luft umgeben ist, kann auch auf den Glas- oder Kunststoffmantel verzichtet werden.

Der erfindungsgemäße Lichtleiter wird bevorzugt in einer Beleuchtungsvorrichtung verwendet. Die Beleuchtungsvorrichtung wird in medizinischen Verfahren und oder Vorrichtung verwendet. Als Beispiels für eine Beleuchtungsvorrichtung seinen in einer nicht vollständigen Aufzählung eine Stirnlampe, ein Endoskop, dass innerhalb eines Patienten verwendbar ist, ein Exoskop, d.h. eine endoskopartige Vorrichtung, die außerhalb eines Patienten verwendbar ist, ein Beleuchtungsschaft oder ein Beleuchtungsstab mit Drehkopf erwähnt. Der erfindungsgemäße Lichtleiter kann grundsätzlich in jeder Beleuchtungsvorrichtung verwendet werden. Der bevorzugte Einsatzbereich des erfindungsgemäßen Lichtleiters liegt jedoch bei medizinischen Vorrichtungen. Der erfindungsgemäße Lichtleiter ist insbesondere derart ausgestaltet, dass er für medizinische Zwecke mehrmals sterilisierbar ist, insbesondere in Autoklaven zur medizinischen Sterilisation.

Nach einem erfmdungsgemäßen Verfahren wird der erfindungsgemäße Lichtleiter mit einem ersten Abschnitt und einem gekrümmten als Verbundabschnitt ausgebildeten zweiten Abschnitt aus lichtleitenden Fasern dadurch hergestellt, dass ein Benetzen von den lichtleitenden Fasern des zweiten Abschnitts mit einem Klebemittel durchgeführt wird, das in einem unausgehärteten, pastösem Zustand ist. Nach dem Einbringen des pastösen Klebemittels wird ein Krümmen des zweiten Abschnitts in eine vorbestimmte Biegungsform durchgeführt wird, wobei das Klebemittel noch in einem pastösen Zustand ist. Erst hiernach wird das Klebemittel bei Aufrechterhaltung der Biegungsform des zweiten Abschnitts ausgehärtet, bis der zweite Abschnitt den formstabilen Verbundabschnitt aus lichtleitenden Fasern und ausgehärtetem Klebemittel mit vorbestimmter Biegungsform bildet. Das Einbringen des Klebemittels in einem pastösen unausgehärteten Zustand hat den Vorteil, dass die Fasern gekrümmt werden können und beim Krümmen bzw. Biegen gegeneinander verschiebbar sind, so dass unnötige Zwangsspannungen vermieden werden können, die ein Brechen der Faser verursachen können. Dass das Klebemittel mit einer pastösen Viskosität in den zweiten Abschnitt zum Ausbilden des Verbundabschnittes eingebracht wird, hat den Vorteil, dass das Klebemittel in diesem Zustand weniger fließfähig ist und es daher zu keinem Abwandern des Klebemittels von der Einbringstelle kommen kann.

Mit pastösem Zustand des Klebemittels wird die Viskosität des Klebmittels beim Auftragen bzw. Einstreichen umschrieben, bei dem das Klebemittel eine derartige Viskosität aufweist, dass einerseits der Klebstoff bzw. das Klebmittel in das Faserbündel einstreichbar und einmassierbar ist, so dass im Wesentlichen jede Faser des Faserbündels mit Klebemittel benetzbar ist, ohne dass Fasern beim Einstreichen/Einmassieren des Klebstoffes brechen, und andererseits der Klebstoff bzw. das Klebmittel eine derartig geringe Fließfähigkeit aufweist, dass der Klebstoff bzw. das Klebmittel nicht durch die Kapillarwirkung in dem Faserbündel abwandern kann.

Nach einem weiteren erfindungsgemäßen Verfahrensschritt zur Herstellung des erfindungsgemäßen Lichtleiters wird vor dem Aufbringen des Klebemittels ein Schrumpfschlauch über einen neben dem zweiten Abschnitt gelegenen Abschnitt der lichtleitenden Fasern gezogen und erst nach dem Benetzen mit dem Klebemittel der lichtleitenden Fasern der Schrumpfschlauch über den mit dem Klebstoff benetzten Abschnitt gezogen. Dies hat den Vorteil, dass der mit dem Klebstoff bzw. Klebemittel benetzte Abschnitt durch den Schrumpfschlauch geschützt ist und insbesondere in einem Biegeverfahren nicht seitlich aus den lichtleitenden Fasern herausquillt und die Biegevorrichtung verschmutzt. Ferner hat der Schrumpfschlauch die Aufgabe, die außen liegenden Fasern zu stabilisieren und Spannungen aufzunehmen und einen Bruch der Fasern zu verhindern.

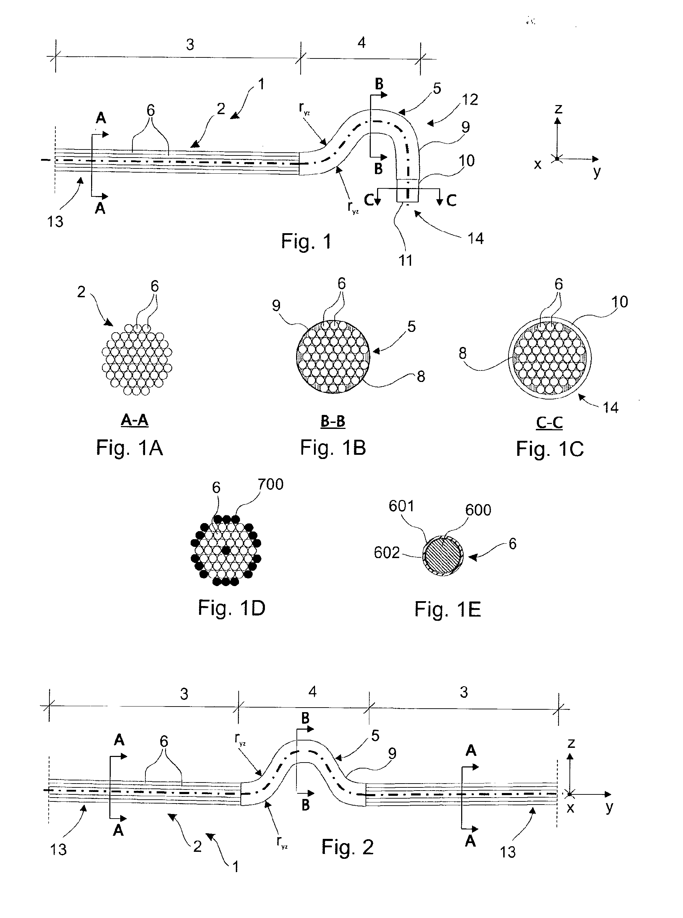

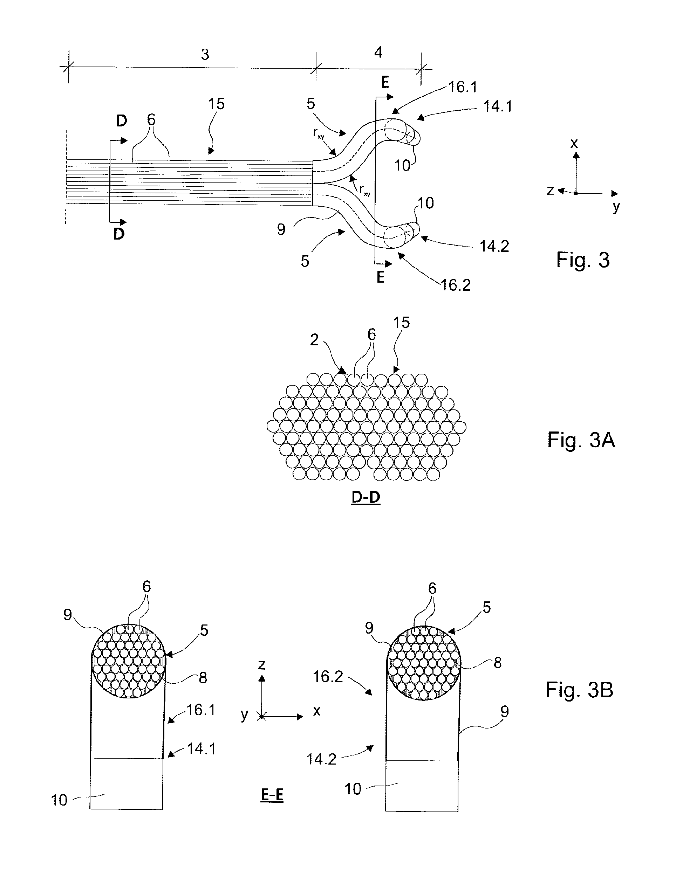

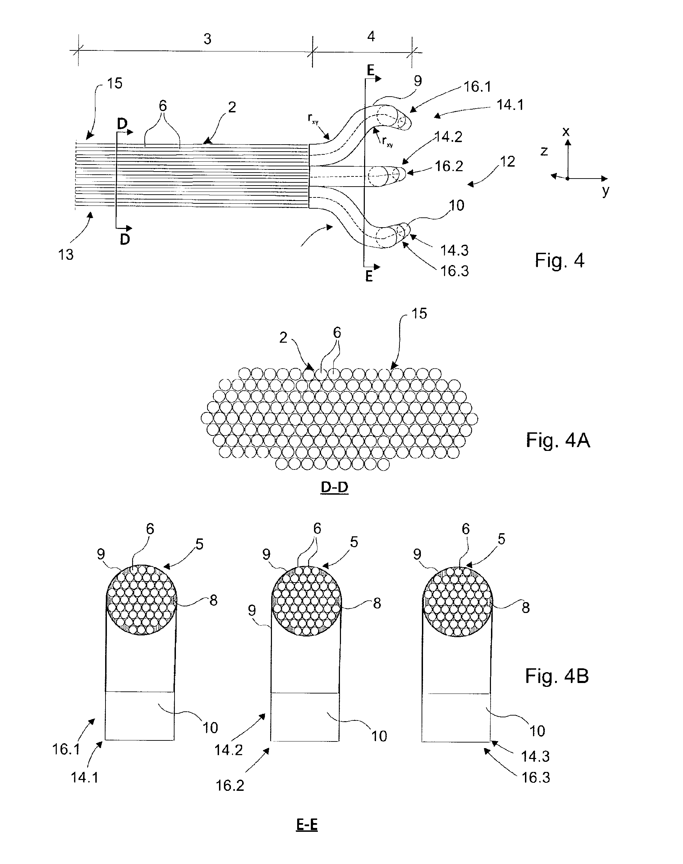

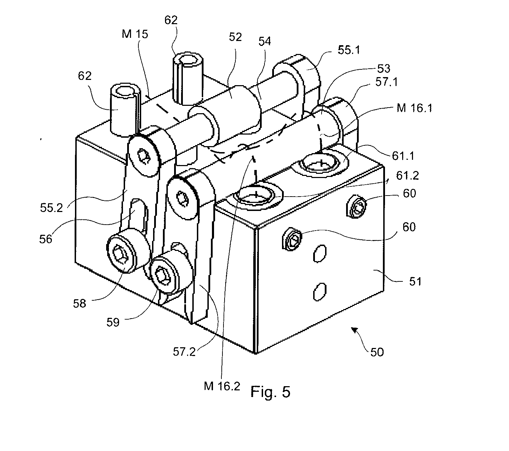

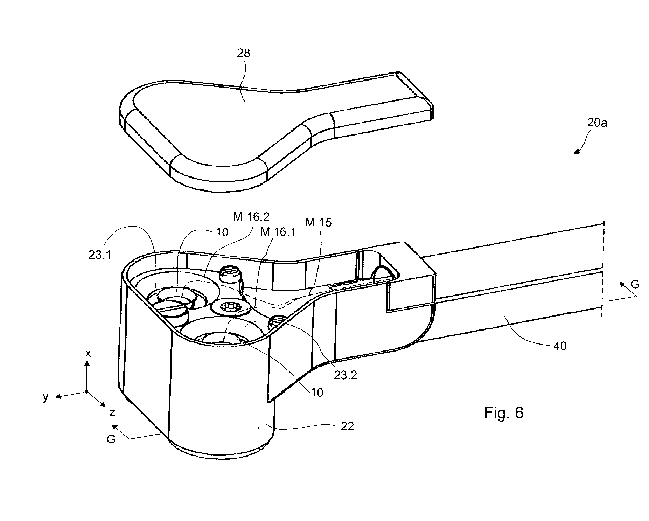

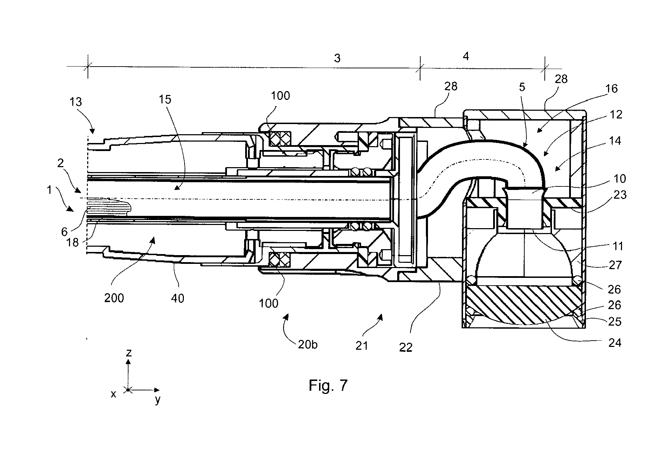

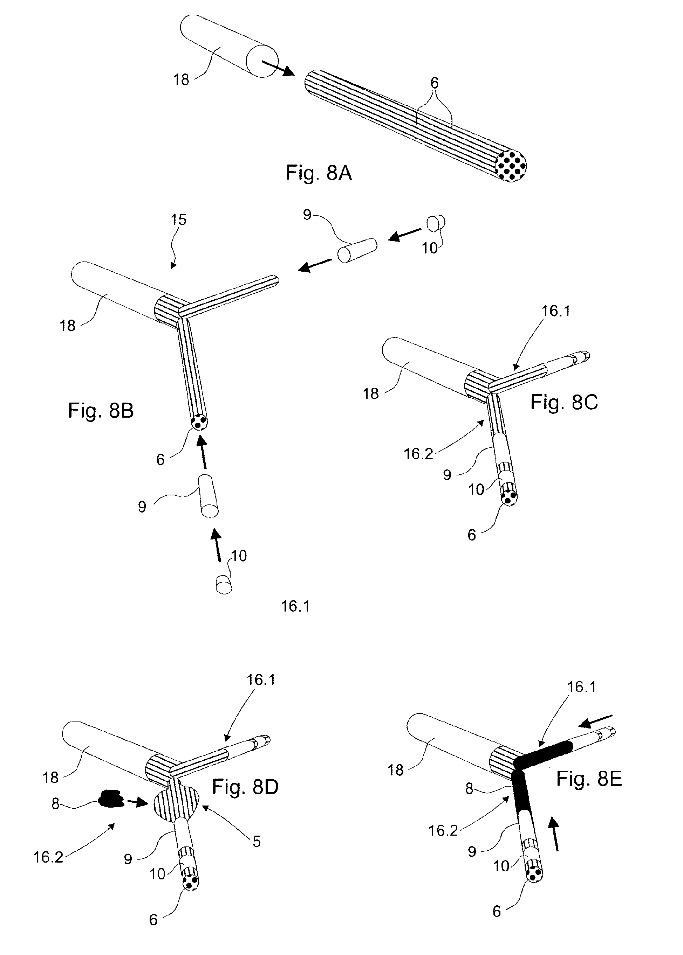

Weitere vorteilhafte Ausgestaltungen der Erfindung, sowie Ausführungsbeispiele hierzu, werden nachstehend in Verbindung mit den beigefügten Zeichnungsfiguren näher erläutert. Funktionsähnliche Bauteile oder Komponenten sind teilweise mit gleichen Bezugszeichen versehen. Die innerhalb der Beschreibung des Ausführungsbeispiels verwendeten Begriffe "links", "rechts", "oben", "unten" beziehen sich auf die Zeichnungsfiguren in einer Ausrichtung mit normal lesbaren Figurenbezeichnungen und Bezugszeichen. Zum besseren Verständnis sind in den nachfolgenden Figuren die jeweiligen Merkmale schematisch und überhöht und nicht maßstäblich dargestellt. Hierbei zeigt:
■ Fig. 1 eine Seitenansicht eines Ausführungsform des erfindungsgemäßen Lichtleiters,
■ Fig. 1A, 1B, und 1C Schnittansichten des in Fig. 1 und 2 gezeigten Lichtleiters gemäß den Schnittführungen in Fig. 1 und 2 in vergrößerter Darstellung,
■ Fig. 1D eine Schnittansicht eines Ausführungsbeispiels des Bündels aus lichtleitenden Fasern des erfindungsgemäßen Lichtleiters in vergrößerter Darstellung,
■ Fig. 1E eine Querschnittansicht einer lichtleitenden Faser, insbesondere einer Glasfaser, zur Verwendung in dem erfindungsgemäßen Lichtleiter, in stark vergrößerter Darstellung,
■ Fig. 2 eine Seitenansicht eines weiteres Ausführungsbeispiel des erfmdungsgemäßen Lichtleiters,
■ Fig. 3 eine Draufsicht auf ein anders Ausführungsbeispiel des erfindungsgemäßen Lichtleiters,
■ Figuren 3A und 3B Schnittansichten des in Fig. 3 gezeigten Lichtleiters gemäß den Schnittführungen in Fig. 3,
■ Fig. 4 eine Draufsichten auf ein weiteres Ausführungsbeispiel des erfindungsgemäßen Lichtleiters,
■ Figuren 4A und 4B Schnittansichten des in Fig. 4 gezeigten Lichtleiters gemäß den Schnittführungen in Fig. 4,
■ Fig. 5 eine Biegevorrichtung zum Krümmen des Verbundabschnittes des in Fig. 3 dargestellten Lichtleiters,
■ Fig. 6 eine perspektivische Ansicht eines Beleuchtungsschaftes für den erfindungsgemäßen Lichtleiter aus Fig. 3, wobei von dem erfindungsgemäßen Lichtleiter nur die Hülsen und die jeweiligen Mittelachsen dargestellt sind,
■ Fig. 7 eine Teilschnittansicht gemäß der Schnittführung G-G des Beleuchtungsschafts aus Fig. 6, wobei der dort gezeigte Beleuchtungsstab zusätzlich einen Drehkopf aufweist, und
■ Figuren 8A bis 8I Verfahrensschritte zum Herstellen eines erfindungsgemäßen Lichtleiters nach Figur 3.

Figuren 1 und 2 zeigen unterschiedliche Ausführungsformen eines erfindungsgemäßen Lichtleiters 1 mit einem ersten, flexiblen Abschnitt 3 und einem zweiten, gekrümmten Abschnitt 4. Der Lichtleiter 1 weist ein Bündel 2 von lichtleitenden Fasern 6 zum Übertragen von Licht einer an einem ersten Endabschnitt 13 des Lichtleiters 1 ankoppelbaren Lichtquelle zu einem zweiten Endabschnitt 12 des Lichtleiters 1 auf. Bei der Lichtquelle (nicht dargestellt) handelt es sich bevorzugt um eine Lichtquelle die Kaltlicht emittieren kann, wie beispielsweise eine Xenon-Lampe, eine Halogen-Lampe oder eine LED. Das Bündel 2 von lichtleitenden Fasern weist mindestens zwei lichtleitende Fasern 6 auf.

Der in Fig.1 gezeigte erfindungsgemäßen Lichtleiter 1, weist an seinem ersten Endabschnitt 13 den ersten flexiblen Abschnitt 3 und an seinem zweiten Endabschnitt 12 den gekrümmten zweiten Abschnitt 4 auf, in denen sich ein Bündel 2 aus lichtleitenden Fasern 6 durchgängig erstreckt. In dem flexiblen ersten Abschnitt 3 sind die lichtleitenden Fasern 6 bevorzugt in ihrer Längsrichtung gegeneinander verschiebbar, d.h. im Wesentlichen in Y-Richtung. In dem ersten Abschnitt 3 ist die Bewegung der einzelnen lichtleitenden Fasern auch in X-Richtung und Z-Richtung nicht eingeschränkt. Die Bewegung der lichtleitenden Fasern 6 in X-Richtung und Z-Richtung kann jedoch durch eine Ummantelung eingeschränkt oder verhindert werden.

In der Fig. 1a ist eine Querschnittsansicht gemäß der Schnittlinie A-A aus der Fig. 1 gezeigt, wobei zum besseren Verständnis die Abstände und Durchmesser der einzelnen lichtleitenden Fasern 6 nicht maßstabgetreu und vergrößert dargestellt sind.

Der gekrümmte zweite Abschnitt 4 des Lichtleiters 1 ist als Verbundabschnitt oder Biegungsabschnitt 5 ausgebildet, der mittels eines ausgehärteten Klebemittels 8 bei Aufrechterhaltung einer Krümmung mit konstanten oder variablen Biegeradius r die lichtleitenden Fasern im Wesentlichen spannungsfrei gegeneinander fixiert. Der Verbundabschnitt oder Biegungsabschnitt 5 weist ein ausgehärtetes Klebemittel 8 auf, das bevorzugt die einzelnen lichtleitenden Fasern 6 des erfindungsgemäßen Lichtleiters 1 derart umgibt, dass das Klebemittel 8 als Stützgerüst wirkt, wobei die gekrümmten lichtleitenden Fasern 6 möglichst spannungsfrei in dem Stützgerüst des Klebemittels 8 angeordnet sind. Das ausgehärtete Klebemittel 8 bildet ein Stützgerüst, in dem die einzelnen lichtleitenden Fasern 6 eingebettet sind. Die im Wesentlichen spannungsfreie Einbettung der lichtleitenden Fasern 6 wird dadurch erzielt, dass das Klebemittel 8 mit einer pastösen Viskosität in den noch ungekrümmten zweiten Abschnitt 4 eingebracht wird und der zweite Abschnitt 4 mit dem noch pastösen Klebemittel 8 in die erforderliche Gestalt gekrümmt wird und erst nach dem Krümmen das Klebemittel 8 ausgehärtet wird. Mit pastösem Zustand des Klebemittels wird die Viskosität des Klebmittels beim Auftragen bzw. Einstreichen umschrieben, bei dem das Klebemittel eine derartige Viskosität aufweist, dass einerseits der Klebstoff bzw. das Klebmittel in das Faserbündel einstreichbar und einmassierbar ist, so dass im Wesentlichen jede Faser des Faserbündels mit Klebemittel benetzbar ist, ohne dass Fasern beim Einstreichen/Einmassieren des Klebstoffes brechen, und andererseits der Klebstoff bzw. das Klebmittel eine derartig geringe Fließfähigkeit aufweist, dass der Klebstoff bzw. das Klebmittel nicht durch die Kapillarwirkung in dem Faserbündel abwandern kann. Dadurch, dass das Klebemittel vor dem Biegen des zu biegenden Verbundabschnittes 5 pastös ist, können sich die einzelnen lichtleitenden Fasern 6 beim Biegevorgang bzw. Krümmungsvorgang in dem pastösen Klebemittel 8 gegeneinander verschieben, ohne dass durch den Biegevorgang Zwangsspannungen an den einzelnen lichtleitenden Fasern aufgebaut werden.

Als Klebemittel 8 werden bevorzugt Klebestoffe angewendet, die vor dem Aushärten pastös sind und ein Verschieben der einzelnen lichtleitenden Fasern während des Biegungsvorgangs erlauben, jedoch nach dem Aushärten ein Stützgerüst bilden, das die einzelnen lichtleitenden Fasern 6 möglichst spannungsfrei in deren Biegung hält. Außerdem weist das Klebemittel 8 vorzugsweise eine Autoklavresistenz auf, so dass eine mehrfache Sterilisation des Verbundabschnitts 5 möglich ist, ohne dass das ausgehärtete Klebemittel 8 durch den Sterilisationsvorgang aufgeweicht oder aufgelöst wird.

Bei dem Klebemittel 8 handelt es sich in der bevorzugten Ausführungsform um einen Zwei-Komponenten Klebstoff, der vor dem Einbringen in den und vor dem Biegen des zweiten Abschnittes 4 auf geeignete Art und Weise gemischt wird, bis die beiden Komponenten mit der erforderlichen Homogenität vermischt sind und die gewünschte pastöse Viskosität zum Einbringen in den zweiten Abschnitt 4 aufweisen.

Fig. 1B zeigt eine Querschnittsansicht gemäß der Schnittführung B-B aus Fig. 1 durch den Verbundabschnitt 5, dem zweiten gekrümmten Abschnitt 4 des erfmdungsgemäßen Lichtleiters 1. Wie aus Fig. 1 und 1B ersichtlich, ist der Verbundabschnitt 5 zur Stabilisierung der außen liegenden lichtleitenden Fasern 6 mit einer Ummantelung 9 aus einem flexiblen und enganliegenden Schlauch versehen, beispielsweise einem Schrumpfschlauch. Die Ummantelung 9 ist aus einem Material, das eine ausreichende Flexibilität aufweist, so dass der Verbundabschnitt zusammen mit dem nicht ausgehärtetem Klebemittel 8 in die erforderliche Gestalt gebogen werden kann, und zusätzlich eine entsprechende Umfassungsfunktion bzw. Stabilisierungsfunktion hat, so dass der von der Ummantelung 9 umgebene zweite Abschnitt 4 in einer im Wesentlichen kreisförmigen Gestalt gehalten wird, insbesondere beim Biegevorgang des zweiten Abschnittes 4. Im gezeigten Ausführungbeispiel ist die Ummantelung 9 bevorzugt aus einem transparenten Schrumpfschlauch aus Polyethylenterephthalat (PET) mit einer sehr dünnen Wandstärke.

In Fig. 1 ist der gekrümmte zweite Abschnitt 4 bzw. Verbundabschnitt 5 in der YZ-Ebene mit einem Biegeradius r_{yz} gezeigt. Der Verbundabschnitt 5 kann nur in der YZ-Ebene einen Biegeradius r_{yz} mit konstanter oder unterschiedlicher Krümmung aufweisen, je nach erforderlichem Verlauf des Verbund- oder Biegungsabschnittes 5. Der Verbundabschnitt 5 kann auch im Wesentlichen geradlinig und ohne eine Krümmung ausgebildet sein. Es kann jedoch je nach Verwendung des erfindungsgemäßen Lichtleiters erforderlich sein, dass der Verbundabschnitt 5 auch in den anderen Ebenen des in Fig. 1 angezeigten Koordinatensystems mit einem entsprechenden Biegeradius r_{xy} und/oder r_{xz} mit konstanter oder unterschiedlicher Krümmung versehen ist.

An dem distalen, lichtabgebenden Endabschnitt 14 des erfindungsgemäßen Lichtleiters 1 ist eine Fassung oder Hülse 10 vorgesehen. Fig. 3C zeigt eine Querschnittsansicht gemäß der Schnittführung C-C aus Fig. 3 durch den lichtabgebenden Endabschnitt 14 des erfindungsgemäßen Lichtleiters 1. Die Hülse 10 ist primär an dem lichtabgebenden Endabschnitt 14 vorgesehen, um ein Nachbearbeiten der Endungen 11 der lichtleitenden Fasern 6 des lichtabgebenden Endabschnittes 14 zu ermöglichen, wie plätten oder polieren, so dass ein Ausfransen des Bündels an der zu bearbeitenden Stelle durch das Polieren verhindert werden kann. Durch das Polieren oder Abschleifen kann die von den Endungen 11 der lichtleitenden Fasern 6 ausgebildete Austrittsfläche in eine ebene, konvexe oder konkave Oberfläche nachbearbeitet werden. Die Hülse oder Fassung 10 ist in dem gezeigten Ausführungsbeispiels aus Metall, insbesondere einem biokompatiblen und autoklavierbaren Metall, das für die Verwendung bei medizinischen Eingriffen geeignet ist. Die Hülse kann auch aus einem biokompatiblen Kunststoff wie Polyetheretherketon (PEEK) hergestellt sein.

Die in dem zweiten, gekrümmten Abschnitt 4 vorgesehenen lichtleitenden Fasern 6 können im gesamten Verbundabschnitt 5 mit demselben Klebemittel versehen sein. Es ist jedoch auch vorstellbar, dass unterschiedliche Klebemittel in den zweiten, gekrümmten Abschnitt eingebracht werden, die nach dem Aushärten unterschiedliche Festigkeiten aufweisen. Wenn zwei unterschiedliche Klebemittel für den Verbundabschnitt 5 verwendet werden, die eine unterschiedliche Festigkeit im ausgehärteten Zustand aufweisen, ist vorzugsweise das in dem lichtabgebenden Endabschnitt 14 im Bereich der Hülse 10 angewendete Klebemittel 8 im ausgehärteten Zustand weniger elastisch als der ausgehärtete Klebstoff 8 in dem gekrümmten Abschnitt 4 zwischen der Hülse 10 und dem flexiblen Abschnitt 3. Mit anderen Worten weist der ausgehärtete Klebstoff 8 in dem gekrümmten Abschnitt 4 zwischen der Hülse 10 und dem flexiblen Abschnitt 3 einen kleineren E-Modul als der ausgehärtete Klebstoff in dem von der Hülse 10 umgebenden lichtabgebenden Endabschnitt 14 auf. Der ausgehärtete Klebstoff 8 in dem gekrümmten Abschnitt 4 zwischen der Hülse 10 und dem flexiblen Abschnitt 3 ist dann ähnlich elastisch wie Gummi oder Kautschuk, wobei der ausgehärtete Klebstoff 8 in dem von der Hülse 10 umgebenden lichtabgebenden Abschnitt 14 eher unelastisch und spröde ist, so dass ein Polieren und Glätten der lichtleitenden Fasern 6 an der Austrittsfläche 11 am lichtabgebenden Abschnitt 14 möglich ist.

Wie aus den Schnittansichten 1B und 1C ersichtlich, sollen die lichtleitenden Fasern 6 jeweils einzeln von dem Klebemittel 8 umgeben sein, so dass jede einzelne lichtleitende Faser 6 von dem ausgehärteten Klebemittel 8 in dem Verbundabschnitt 5 und in dem lichtabgebenden Endabschnitt 14 spannungsfrei fixiert ist.

Fig. 2 zeigt ein weiteres erfindungsgemäßes Ausführungsbeispiel des erfindungsgemäßen Lichtleiters 1, wobei der Verbundabschnitt 5 mit Schrumpfschlauch 9 nicht an einem lichtabgebenden Endabschnitt des Glasfaserbündels vorgesehen ist, sondern an einem Zwischenabschnitt eines ersten, flexiblen Abschnittes 3.

Die lichtleitenden Fasern 6 im Bündel 2 des in den Figuren gezeigten erfindungsgemäßen Lichtleiters 1 sind bevorzugt Glasfasern 6, insbesondere Glasfasern 6 mit einem Glaskern 600 mit einem Durchmesser von etwa 20 bis 500 µm, der mit einem Glasmantel 601 mit einer etwa konstanten Dicke von etwa 2,5 µm umgeben ist.

In Figur 1E ist ein Querschnitt einer lichtleitenden Glasfasern 6 gezeigt, die in den erfindungsgemäßen Lichtleitern verwendet wird. Die Glasfaser 6 weist einen Glaskern 600 auf, der von einem Glasmantel 601 umgeben ist. Der Glaskern 600 ist aus einem zum lichtübertragen geeigneten Glas ausgebildet, wobei der Glasmantel 602 von einem Glas oder Kunststoff ausgebildet ist, der einen Brechungsindex hat, der in etwa dem Brechungsindex der Luft entspricht. Das den Glaskern 600 umgebenden Mantel-Material 602, sollte außerdem folgende Bedingungen erfüllen: das verwendete Material des Mantels 601 darf sich beim Herstellen der lichtleitenden Faser 6 nicht mit dem Glaskern 600 vermischen, so dass eine klare Trennschicht 602 zwischen dem Mantel 601 und dem Glaskern 600 besteht. In der Trennschicht 602 zwischen Glaskern 600 und Mantel 601 dürfen außerdem keine Zwischenräume vorhanden sind, da die Zwischenräume sich negativ auf die Lichttransmission auswirken können. Außerdem muss das zu verwendende Glas bzw. Kunststoff biokompatibel und autoklavierbar sein.

Es ist auch möglich, dass auch einzelne oder mehrere der lichtleitenden Fasern 6 vollständig, d.h. der Kern und der Mantel aus einem lichtleitenden Kunststoff hergestellt sind, insbesondere die lichtleitenden Fasern 6, die einer besonders großen Biegung oder Krümmung in dem Bündel 2 unterworfen werden, wie beispielsweise die lichtleitenden Fasern nahe am Mittelpunkt des etwa kreisförmigen Bündels 2 aus lichtleitenden Fasern 6. In der Figur 1D sind exemplarisch die aus Kunststoff ausgebildeten lichtleitenden Fasern mit 700 angezeigt und mit schwarz ausgefüllt. Wie hieraus ersichtlich, sind insbesondere die lichtleitenden Fasern 700 an der Außenumfangsfläche und am Mittelpunkt aus einem lichtleitenden Kunststoff ausgebildet. Grundsätzlich können die Faser 6, die einer besonders starken Krümmung ausgesetzt sind aus einem geeigneten, elastischen Kunststoff hergestellt sein, während die weniger gekrümmten Fasern 6 aus Glas hergestellt sein können.

In den Figuren 3 und 4 sind weitere Ausführungsbeispiele des erfindungsgemäßen Lichtleiters 1, insbesondere der Ausgestaltung des Biegungsabschnittes bzw. Verbundabschnittes 5 dargestellt.

Der Verbundabschnitt 5 kann als einstrangiger Abschnitt mit einem im Wesentlichen konstanten Flächenquerschnitt, wie in Fig.1 und den Schnittansichten aus Figuren 1A bis 1C gezeigt, oder auch als mehrstrangiger Abschnitt ausgebildet sein, wie in Figuren 3 und 4 gezeigt.

In Figuren 3 und 4 sind Ausführungsbeispiele der Aufteilung des Hauptstrangs 15 des Bündels 2 aus lichtleitenden Fasern 1 in unterschiedliche Nebenstränge 16.1, 16.2, 16.3 dargestellt. Die in den Figuren 3 und 4 gezeigte Draufsicht entspricht einer Ansicht auf die XY-Ebene gemäß dem in Fig.3 gezeigten dreidimensionalen Koordinatensystem.

Fig.3 zeigt den erfindungsgemäßen Lichtleiter 1 mit einem Hauptstrang 15 mit einem vorbestimmten Flächenquerschnitt A, der sich Y-förmig in zwei Nebenstränge 16.1 und 16.2 mit jeweils einen vorbestimmten Flächenquerschnitt B1 und B2 in einen ersten Nebenstrang 16.1 und in einen zweiten Nebenstrang 16.2 erstreckt. Die Flächenquerschnitte B1 und B2 sind bevorzugterweise im Wesentlichen identisch. Es ist jedoch auch möglich, die Nebenstränge B1 und B2 mit jeweils unterschiedlichen Flächenquerschnitten zu versehen. Die Nebenstränge sind in dem Verbundabschnitt 5 jeweils in der XY-Ebene mit gemäß den in Fig.3 angezeigten Biegeradius r_{xy} gekrümmt und in den anderen Ebenen mit den Biegeradien r_{xz} und r_{yz} gekrümmt.

Die lichtabgebenden Endabschnitte 14.1 und 14. 2 sind in der yz-Ebene korrespondierend zu dem in der Fig. 1 gezeigten Biegeradius r_{xz} gekrümmt.

Bevorzugterweise sind die Biegungsverläufe der Nebenstränge 16.1 und 16.2 in den jeweiligen Ebenen des X-Y-Z-Koordinatensystems derart, dass der von dem Verbundabschnitten 5 gebildete zweite, gekrümmte Abschnitt 4 für eine Anwendung in dem in den Figuren 6 und 7 gezeigten Beleuchtungsstab 20 geeignet ist. Der gekrümmte Abschnitt 4 und der die Biegung aufrechterhaltende Verbundabschnitt 5 können jedoch auch andere, nicht in den Figuren gezeigte, Biegungsverlaufe haben. Die Anfertigung des gekrümmten zweiten Abschnittes 4 mittels des Verbundabschnittes 5 ist derart ausgelegt, dass der gekrümmte zweite Abschnitte 4 und auch der lichtabgebende Endabschnitt 14 je nach Anwendungsfall an die geforderten Bedingungen bzw. Gehäusegeometrien anpassbar ist. Der in Fig. 3 dargestellte Lichtleiter 1 ist in dem in Figuren 6 und 7 gezeigten Beleuchtungsstab 20 verwendbar ist.

In Fig. 4 ist ein mögliches Ausführungsbeispiel des erfindungsgemäßen Lichtleiters 1 mit einem Hauptstrang 15 mit einem vorbestimmten Flächenquerschnitt A gezeigt, der sich dreizackartig in drei Nebenstränge 16.1 bis 16.3 mit jeweils einemvorbestimmten Flächenquerschnitt B1 bis B3 erstreckt. Die drei Nebenstränge 16.1 bis 16.3 bilden den gekrümmten Abschnitt 4 aus, der von dem ausgehärteten Klebemittel in dem jeweiligen Verbundabschnitt 5 in der gekrümmten Gestalt gehalten wird. Der einzelne Nebenstrang 16.1, 16.2 und 16.3 ist in den jeweiligen Ebenen des X-Y-Z-Koordinatensystems mit einer entsprechenden Krümmung und Biegeradien r_{xy}, r_{yz} und/oder r_{xz} versehen, so dass die lichtabgebenden Endabschnitte 14.1 bis 14.3 in die entsprechenden Aufnahmen einer Beleuchtungsvorrichtung einsetzbar sind. Falls erforderlich, können die jeweiligen Nebenstränge auch in unterschiedliche Richtungen zeigen, je nachdem, wie die Aufnahmen für die lichtabgebenden Endabschnitte 14 in der Beleuchtungsvorrichtung angeordnet sind.

In Fig. 5 ist eine Biegevorrichtung gezeigt, die in dem Verfahren zum Herstellen des erfindungsgemäßen Lichtleiters 1, wie in Fig. 3 gezeigt, verwendet wird.

Die Biegevorrichtung 50 weist ein Gehäuse 51 auf, das vorzugsweise aus einem Material hergestellt ist, das die beim Aushärten des Klebemittels 8 auftretenden Drücke und Temperaturen in einem Ofen standhalten kann, ohne sich wesentlich zu verformen.

Die Biegevorrichtung zum Herstellen des erfindungsgemäßen Lichtleiters weist eine Andrück- oder Biegewalze 52 und eine Umlenkwalze 53 auf. Die Andrück-und Biegewalze 52 ist über einen Querträger 54 an dem Gehäuse 51 mittels eines ersten und zweiten Schwenkarms 55.1, 55.2 an dem Gehäuse befestigt, wobei der erste und zweite Schwenkarm 55.1, 55.2 jeweils eine Ausnehmung 56 aufweist, die ein Höhen- und Schwenkverschieben des ersten und zweiten Schwenkarms 55.1, 55.2 erlaubt. Die erforderliche Position des ersten und zweite Schwenkarms 55.1, 55.2 und damit die Position des Querträgers 54 ist mittels einer Feststellschraube 59, die in die Ausnehmung 56 des jeweiligen Schwenkarms 55.1, 55.2 eingreift, hier in der Form einer Inbusschraube, fixierbar.

Die Umlenkwalze 53 weist einen im Querschnitt im Wesentlichen kreisförmigen Querträger auf, der einen vorbestimmten Durchmesser aufweist und ebenfalls mit einem dritten und vierten Schwenkarm 57.1, 57.2 an dem Gehäuse 51 höhen- und schwenkverstellbar angebracht ist. Die gewünschte Position des dritten und vierten Schwenkarms 57.1, 57.2 und damit die Position der Umlenkwalze 53 ist ebenfalls mittels einer Feststellschraube 59, die in die Ausnehmung 56 des jeweiligen Schwenkarms 57.1, 57.2 eingreift, hier in der Form einer Inbusschraube, fixierbar. Der Querschnitt der Umlenkwalze 53 kann je nach erforderlicher Krümmung des Verbundabschnittes 5 des Lichtleiters 1 rechteckig, vieleckig, oder elliptische ausgebildet sein.

Benachbart zu der Umlenkwalze 53 sind an der Oberseite des Gehäuses 51 der Biegevorrichtung 50 Aufnahmebecher 61.1 und 61.2 für die lichtabgebenden Endabschnitte 14.1 und 14.2 vorgesehen. Die Aufnahmebecher 61.1 und 61.2 sind in dem Gehäuse 51 in Bohrungen auswechselbar gelagert und mittels Madenschrauben 60 fixierbar.

Zur Ausbildung der erforderlichen Biegung oder Krümmung des zweiten gekrümmten Abschnittes 4 des Lichtleiter 1 kann an Stelle oder in Kombination mit der Biegevorrichtung auch eine formgebende Schale (nicht dargestellt) verwendet werden, in der eine Ausnehmung oder Kavität ausgebildet ist, mit der das Faserbündel in die erforderliche Biegung durch Einlegen des Faserbündel gebracht wird. Die Schale ist entweder an sich formstabil, so dass die Faserbündel sich an der Kavitäts-Wandung der Schalung abstützen können und deren vorgegebene Biegung einnehmen, kann aber auch derart flexibel ausgebildet sein, das die Schale in Kombination mit der Biegevorrichtung verwendbar ist, wobei die Schale sowohl formgebende Eigenschaften aufweisen kann als auch derart flexibel ausgestaltet ist, dass die Schale von der Biegevorrichtung in die erforderliche Gestalt bzw. Biegung gebogen werden kann. Bevorzugt ist die Schale aus einem Material, dass keinen festen Kontakt mit dem Klebmittel eingehen kann. Bevorzugterweise ist die Schale aus Teflon hergestellt, wie auch die Aufnahmebecher 61.1, 61.2.

Ferner sind an der Oberseite des Gehäuses 51 der Biegevorrichtung 50 benachbart zu dem Querträger 54 der Andrück- bzw. Biegewalze 52 Führungsstifte 62 vorgesehen, die den Hauptstrang 15 des erfindungsgemäßen Lichtleiters beim Biegevorgang an Ort und Stelle halten. Die Position des Lichtleiters 1 ist in der Biegevorrichtung 50 mittels der Mittelachsen M15, M16.1 und M16.2 der jeweiligen Stränge angedeutet.

Die Herstellung des erfindungsgemäßen Lichtleiters 1, wie in Fig. 3 gezeigt, erfolgt wie nachfolgend aufgeführt, wobei die Verfahrensschritte in den Figuren 8A bis 8F schematisch dargestellt sind.

Zunächst wird ein Bündel 2 aus lichtleitenden Fasern 6 zum Ausbilden des Hauptstranges 15 derart zusammengefasst, vorzugsweise durch Abbinden mittels eines Bindfadens, so dass ein Faserbündel 2 mit einem vorbestimmten Flächenquerschnitt A von ungefähr 20 mm² gebildet wird.

An dem proximalen Endabschnitt 12 des hierdurch erhaltenen Hauptstrangs 15 kann ein passender Schrumpfschlauch 18 aufgezogen werden, wie in Fig. 8A dargestellt.

Im nächsten Schritt werden zum Ausbilden der Y-förmig abzweigenden Nebenstränge 16.1 und 16.2 von dem Hauptstrang 15 zwei Faserbündle etwa mit gleich großem Flächenquerschnitt B2, also etwa 10 mm², ausgebildet, indem die noch lose miteinander verbundenen lichtleitenden Fasern 6 entsprechend aufgeteilt werden. Auf die lichtabgebenden Endabschnitte 14.1, 14.2 der Nebenstränge 16.1 und 16.2 wird dann ein Schrumpfschlauch 9 aufgeschoben. Hiernach wird auf die Endabschnitte 14.1, 14.2 jeweils eine Hülse 10 aufgesteckt, wobei ein Bündelabschnitt vorbestimmter Länge aus der Hülse hervorsteht, wie in Figuren 8B und 8C gezeigt.

Bevor die Schrumpfschläuche 9 der Nebenstränge 16.1 und 16.2 vollständig über den Bereich geschoben werden, der als Verbundabschnitt 5 ausgebildet werden soll, wird über eine vorbestimmte Länge, vorzugsweise etwa 25 mm, in das jeweilige Bündel aus lichtleitenden Fasern 6 der Nebenstränge 16.1 und 16.2 ein Klebemittel 8 (siehe Fig. 8D) in einem pastösen Zustand eingebracht, so dass möglichst jede Faser 6 des Bündels mit Klebemittel 8 benetzt wird. Die Länge des mit Klebstoff zu versehenden Verbundabschnittes 5 kann je nach erforderlicher Krümmung auch kürzer oder länger sein. Das Benetzen bzw. Einbringen des pastösen bzw. fließfähigen Klebemittels 8 findet bevorzugt derart statt, dass der mit dem Klebemittel 8 zu benetzende Abschnitt 5 fächerartig aufgefächert wird, wie in Fig. 8D angedeutet, und das Klebemittel mittels eines Einbring-Werkzeuges auf das aufgefächerte Faserbündel 2 aufgetragen und einmassiert wird, wobei möglichst alle lichtleitenden Fasern 6 mit dem Klebemittel 8 benetzt werden sollen.

Der Klebstoff bzw. das Klebemittel 8 wird vorzugsweise mittels eines Einbring-Werkzeuges derart einmassiert, dass möglichst jede einzelne Faser 6 mit dem pastösen Klebemittel versehen ist. Bei dem verwendeten Einbring-Werkzeug ist zu beachten, dass das pastöse Klebemittel einfach in das Bündel 2 von lichtleitenden Fasern eingebracht werden kann. Als geeignetes Werkzeug zum Einmassieren des Klebemittels haben sich hierbei insbesondere Holzstäbchen oder Pinsel herausgestellt.

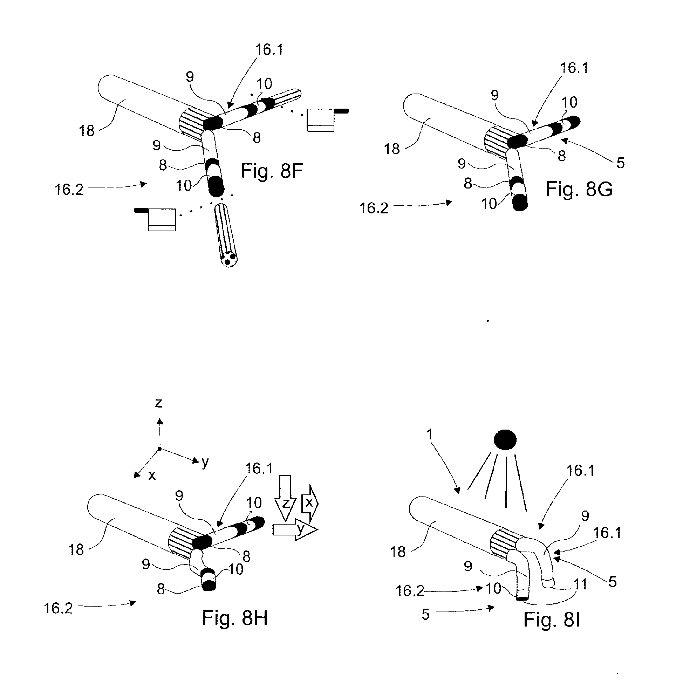

Nach dem Einmassieren werden dann die Schrumpfschläuche der Nebenstränge 16.1, 16.2 über die Bereiche des Faserbündels gezogen, in die das Klebemittel eingebracht worden ist, wie in Fig. 8E angedeutet, wobei die Hülsen entsprechend mitgeführt werden. Die mit dem Klebemittel 8 versehenen Fasern 6 bilden nun einen Verbundabschnitt 5 aus. Dann wird ein möglicher Überstand aus nicht mit Klebemittel versehenen lichtleitenden Fasern 6 am lichtabgebenden Endabschnitt 14 an der Fassung oder Hülse 10 mit einer geeigneten Schneidvorrichtung abgetrennt, wie in Fig. 8F mit dem Beil-Symbol angedeutet, so dass ein steckerartiger Anschluss ausgebildet ist, der in die Aufnahmen 61 der Biegevorrichtung 50 (siehe Fig. 5) bzw. eine Aufnahme 23 eines Beleuchtungsstabes (siehe Figuren 6 und 7) einsetzbar ist und um den in den Nebensträngen 16.1 und 16.2 gebildeten Verbundbereich 5 wie in Fig. 8G zu erzielen. Dann wird der mit dem noch pastösen Klebemittel versehene Verbundabschnitt 5 mit Hilfe einer Biegevorrichtung 50 oder einer entsprechenden Schale in die geforderte Biegeform gebogen, wie in Fig. 8H schematisch angedeutet. Um den Klebstoff auszuhärten, so dass der Klebstoff die geforderte Krümmung aufrechterhält, wird der Lichtleiter 1 zusammen mit der Biegevorrichtung durch Temperaturerhöhung ausgehärtet, wie in Fig. 8I angedeutet. Dies kann in einem geeigneten Ofen durchgeführt werden.

In Fig. 5 ist die Biegevorrichtung 50 dargestellt, mit der der mit dem pastösen Klebemittel 8 versehene Verbundabschnitt in die erforderliche Krümmung gebracht und gehalten werden kann, bis das Klebemittel ausgehärtet ist und als Stützgerüst wirkt und die Krümmung der lichtleitenden Fasern 6 aufrecht erhalten kann.

Wenn die in Fig. 5 dargestellte Biegevorrichtung verwendet wird, werden zum Erzielen der Krümmung der Fasern 6 jeweils die mit der Hülse 10 versehenen lichtabgebenden Endabschnitte 14 in die jeweiligen Aufnahmebecher 61 der Biegevorrichtung 50 eingesetzt. Zum besseren Übersichtlichkeit sind nur die Mittelachsen M15, M16.1 und M16.2 des Lichtleiters 1 dargestellt. Der mit dem unausgehärteten Klebemittel 8 versehene Verbundabschnitt 5 und wird auf die Umlenkwalze 53 gelegt und der an den Verbundabschnitt 5 angrenzende flexible Abschnitt 3 wird unter die Andrück- und Biegewalze 52 fixiert. Die Andrück- und Biegewalze 52 drückt dann die Nebenstränge 16.1 und 16.2 in die geforderte Biegung gegen die Umlenkwalze 53 bzw. nach unten in Richtung Gehäuse 51, wobei der Hauptstrang 15 des Bündels 2 von Führungsstiften 62 an Ort und Stelle gehalten wird. Wesentlich beim Krümmen der Nebenstränge 16.1 und 16.2 ist, dass das Klebemittel noch in einem unausgehärteten Zustand ist und ein Verlagern der einzelnen lichtleitenden Fasern 6 gegeneinander beim Krümmen die gewünschte Form erlaubt, so dass keine Zwangsspannungen in den lichtleitenden Fasern 6 ausgebildet werden.

Das Klebemittel 8 wird dann, nachdem die lichtleitenden Fasern 6 zusammen mit dem pastösen, unausgehärteten Klebemittel 8 in die gewünschte Biegung gebracht worden sind, in einem Ofen durch Temperaturerhöhung ausgehärtet. Nachdem der Klebstoff bzw. das Klebemittel 8 ausgehärtet sind, werden die lichtleitenden Fasern 6 von dem ausgehärteten Klebemittel spannungsfrei in dem Verbundabschnitt 5 gehalten, wobei beim Aushärtevorgang gleichzeitig die Schrumpfschläuche am Haupt- und Nebenstrang die jeweiligen Faserbündel fest umgeben.

Üblicherweise haben die als Klebemittel verwendeten Klebstoffe im unausgehärteten Zustand und vor dem Einbringen in das Faserbündel thixotrope Eigenschaften, d.h. die Viskosität oder Fließfähigkeit des Klebstoffes kann durch mechanische Einwirkungen wie Rütteln oder Schütteln des beispielsweise zu viskosen Klebstoffs auf die gewünschte pastöse Viskosität eingestellt werden. Bevorzugt weist das Klebmittel beim Einbringen eine Viskosität auf, die vergleichbar der von Zahnpasta bei Raumtemperatur ist. Als Klebstoff werden bevorzugt 2-Komponenten Klebstoffe verwendet, die vor dem Einbringen in das Faserbündel 2 derart gemischt werden, dass eine homogene Mischung der beiden Komponenten vorliegt.

In Fig. 6 ist eine Beleuchtungsvorrichtung, insbesondere ein Beleuchtungsschaft 20a, gezeigt, in der die in Fig. 3 dargestellte Ausführungsform des erfindungsgemäßen Lichtleiters 1 mit einem y-artig sich verzweigenden Verbundabschnitt 5 angewendet werden kann. Die gezeigte Beleuchtungsvorrichtung ist ein Beleuchtungsschaft 20a, der für eine Verwendung bei medizinischen Eingriffen geeignet ist. Der Beleuchtungsschaft 20a ist aus Materialien hergestellt, die ein mehrfaches medizinisches Sterilisieren, insbesondere in einem Autoklaven widerstehen können.

In Fig. 6 ist eine perspektivische Darstellung des Beleuchtungsschaftes 20a mit einem formstabilen Gehäuse 22 und einem flexiblen Kabel 40 gezeigt, Das Gehäuse 22 ist zur Aufnahme der lichtabgebenden Endabschnitte 14.1 und 14.1 des erfindungsgemäßen Lichtleiters 1 nach Fig. 3, insbesondere der daran angebrachten Hülse 10, und einer optischen Linse 24 (siehe Fig. 7) ausgebildet. Zur besseren Übersicht ist die oberer Abdeckung 28 des Beleuchtungsschaft 20a in einer abgehobenen, demontierten Position gegenüber dem Gehäuse 22 dargestellt und von dem erfindungsgemäßen Lichtleiter nach Fig. 3 sind nur die Hülsen 10 und der Verlauf der Mittelachsen M15, M16.1, M16.2 des Hauptranges 15 und der Nabelstränge 16.1 und 16. 2 angedeutet. In dem flexiblen Kabel 40 ist der flexible, zweite Abschnitt 4 des Bündels 2 aus lichtleitenden Fasern 6, bevorzugt der Hauptstrang 15 derart untergebracht, dass das Bündel 2 von dem Kabel flexibel gehalten wird. Zur Aufnahme der Hülse 10 bzw. der lichtabgebenden Endabschnitte 14 sind in dem Gehäuse 22 Aufnahmen 23.1 und 23.2 vorgesehen.

In dem Kabel 40 ist der Hauptstrang 15 bzw. der flexible Abschnitt 3 des erfindungsgemäßen Lichtleiters 1 angeordnet. In dem formstabilen Gehäuse 22 des Beleuchtungsstabes 22 sind der erste und der zweite Nebenstrang 16.1 und 16.2 untergebracht.

In dem in Fig. 3 dargestellten Ausführungsbeispiel des erfindungsgemäßen Lichtleiters 1 sind die jeweiligen Nebenstränge 16.1 und 16.2 an ihrem lichtabgebenden Endabschnitt 14.1, 14.2 jeweils mit einer Hülse 10.1, 10.2 versehen, die in einer korrespondierenden Aufnahme 23.1, 23.2 des Gehäuses 22 des in Fig. 6 gezeigten Beleuchtungsstabes 20b des Drehkopfes 21 einsetzbar sind und mit entsprechenden Maßnahmen dort arretierbar sind. Beispielsweise können die Hülse 10 des Lichtleiters 1 an den Aufnahmen 23.1, 23.2 des Gehäuses 22 des Beleuchtungsstabes 20b durch ein entsprechendes Klebemittel fixiert werden.

In Fig. 7 ist in einer Teilschnittansicht eines anderen Ausführungsbeispiels des erfindungsgemäßen Lichtleiters 1, wie in Fig. 1 gezeigt, zusammen mit einem Beleuchtungsstab 20b gezeigt, der sich von dem in Fig. 6 gezeigten Drehschaft darin unterscheidet, das der Beleuchtungsstab einen Drehkopf aufweist, wobei die Schnittführung G-G in Fig. 6 für den Beleuchtungsschaft 20a zum besseren Verständnis verwendet wird. Der in Fig.6 gezeigte Beleuchtungsschaft 20a unterscheidet sich zusätzlich vom dem in Fig.7 gezeigten Beleuchtungsstab 20b darin, dass der Drehkopf 21 zur Aufnahme des in Fig. 1 gezeigten einstrangingen Lichtleiters 1 ausgebildet ist.

Der in Fig. 7 dargestellte Beleuchtungsstab 20b weist den Drehkopf 21 auf, der gegenüber einem Kabel 40 um die Mittelachse M drehbar gelagert ist. Der Drehkopf 21 weist ein Gehäuse 22 auf. Das Gehäuse 22 weist eine Aufnahme 23 für die Hülse 10 des lichtabgebenden Endabschnittes 14 des Lichtleiters 1 auf. In dem Gehäuse 22 ist auch eine optische Linse 24 untergebracht. Die optische Linse 24 kann beispielsweise eine Sammellinse oder eine Zerstreuungslinse oder jedes andere geeignete optische Vorrichtung sein. In der Fig. 7 ist in der Halterung 26 eine plan-konvexe Sammellinse 24 gezeigt, wobei die plane Fläche der Sammellinse 24 dem lichtabgebenden Endabschnitt 14 des Lichtleiters 1 zugewandt ist. Die optische Linse ist zwischen Dichtungsringen 26 angeordnet. Zwischen der Aufnahme 23 und der optischen Linse 24 ist eine Abstandhalter 27 vorgesehen. Der Abstandshalter 27 ist erforderlich, um die optische Linse 24 mit einem geeigneten Abstand von der Endungsfläche 11 des Lichtleiters 1 anordnen zu können.

Der erfindungsgemäße Lichtleiter 1 ist mit der Hülse 10 an dem lichtabgebenden Endabschnitt 14, der von dem ersten gekrümmten Endabschnitt 12 ausgebildet ist, in einer Aufnahme 23 des Gehäuses 22 des Beleuchtungsstabes 20 eingesetzt. Vorzugsweise ist die Aufnahme 23 derart ausgebildet, dass die Hülse 14 des Lichtleiters 1 in der Aufnahme 23 durch Presspassung gehalten wird. Es ist auch möglich, die Hülse 10 mittels eines Klebstoffes in der Aufnahme 23 zu fixieren. Außerdem ist es möglich, die Hülse 10 mittels eines lösbaren Verbindungsmittels, wie einer Madenschraube (nicht dargestellt), die im Bereich der Aufnahme 23 vorgesehen ist, zu fixieren.

Wie aus Fig. 7 ersichtlich, sind in dem Gehäuse des Beleuchtungsstabes 20 keine zusätzlichen Halte- oder Stützvorrichtungen für den Lichtleiter 1 im Bereich des Verbundabschnittes 5 erforderlich, die die Herstellung des Gehäuses 22 aufwendiger und teurer machen würden. Der formstabile Verbundabschnitt 5 des in dem Gehäuse 22 eingesetzten Lichtleiters 5 erleichtert nicht nur den Einbau in das Gehäuse, sondern bietet auch einen Bruch- und Knickschutz für die in dem gekrümmten Abschnitt 4 und in dem Verbundabschnitt 5 eingebetteten lichtleitenden Fasern 6.

Die lichtleitenden Fasern 6 des in dem Beleuchtungsstab 20b verwendeten Bündels 2 des Lichtleiters 1 bestehen aus Glasfasern, insbesondere Glasfasern mit einem Glaskern 600 mit einem Durchmesser von etwa 70 µm, der mit einem Glasmantel 601 mit einer Dicke von etwa 2,5µm umgeben ist (siehe Fig. 1E). Der Beleuchtungsstab 20b mit Drehkopf 21 ist in der bevorzugten Ausführungsform für die Verwendung des in Figur 1 gezeigten einstrangigen Lichtleiters 1 ausgebildet und weist nur eine Aufnahme 23 auf. Der Drehkopf 21 kann aber auch mit zwei Aufnahmen 23.1, 23.2 versehen sein, so dass der in Figur 3 gezeigte zweistrangige Lichtleiter 1 verwendet werden kann.

Das Kabel 40 ist mit dem Drehkopf 21 des Gehäuses 22 fluiddicht über Dichtungen 100 verbunden, so dass eine Torsionsbewegung des Drehkopfes 21 um die Mittelachse M möglich ist.

In dem Kabel 40 ist der flexible, erste Abschnitt 3 des erfindungsgemäßen Lichtleiters 1 derart untergebracht, dass die einzelnen lichtleitenden Fasern 6 die Möglichkeit haben, wenigstens im ersten Abschnitt in XY- und Z-Richtungen sich gegeneinander zu verlagern, insbesondere dann, wenn das Kabel 40 zum Ausrichten des Drehkopfes 21 verformt wird.

Das Kabel 40 ist vorzugsweise aus einem flexiblen, also biegbaren Schutzmaterial, das bei medizinischen Vorrichtungen mit einem flexiblen Versorgungsschlauch, wie Endoskopen, üblich ist. Der erfindungsgemäße Lichtleiter 1 wird an Ort und Stelle mittels der Aufnahme 23 innerhalb des Gehäuses gehalten. Bevorzugt ist das Bündel 2 des erfindungsgemäßen Lichtleiters 1 in dem ersten flexiblen Abschnitt 3 mit einer Ummantelung 18 versehen. Die Ummantelung 18 ist ein Schutzschlauch 18 aus einem Material, das besonders reibungsarm ist.

In der Kavität 200 zwischen der Ummantelung 18 und dem Mantel des Kabel 40 ist bevorzugt eine Spiralwendel vorgesehen, die den Bewegungsspielraum des Bündels aus lichtleitenden Fasern 6 einschränkt, um den Bruch einzelner lichtleitender Fasern 6 beim Betätigen und Handhaben des Beleuchtungstabes zu erleichtern.

Der proximal zu einer Lichtquelle (nicht dargestellt) angeordnete Endabschnitt 13 weist eine Ankoppelvorrichtung (nicht gezeigt) auf. Mit der Ankoppelvorrichtung des Beleuchtungstabes 20 werden die einzelnen lichtleitenden Fasern derart mit der Licht emittierenden Lichtquelle verbunden, dass das von der Lichtquelle emittierte Licht von dem proximalen Endabschnitt 13 des Lichtleiters hin zu dem zu der Lichtquelle angeordneten distalen zweiten Endabschnitt 3 übertragen und aus dem lichtabgebenden Endabschnitt 14 austreten kann. Die Lichtquelle emittiert bevorzugt Kaltlicht. Als Lichtquelle wird bevorzugt eine Xenon-Lichtquelle verwendet. Es ist jedoch auch vorstellbar, dass die Lichtquelle eine Halogenlichtquelle oder eine LED-Lichtquelle ist.

Der erfindungsgemäße Lichtleiter ist nicht nur auf die Verwendung in den oben beschriebenen Beleuchtungsstab beschränkt. Vielmehr kann der erfindungsgemäße Lichtleiter in anderen, insbesondere medizinischen, Beleuchtungsvorrichtungen verwendet werden, wie Endoskope, Exoskope oder Stirnlampen.

### Bezugszeichenliste

| Nr. | Merkmal | Nr. | Merkmal |
|---|---|---|---|
| 1 | Lichtleiter | 20a/b | Beleuchtungsschaft/Beleuchtungsst ab |
| 2 | Bündel | 21 | Drehkopf |
| 3 | flexibler, erster Abschnitt | 22 | Gehäuse |
| 4 | gekrümmter, zweiter Abschnitt | 23 | Hülsen-Aufnahme |
| 5 | Verbundabschnitt, Biegungsabschnitt | 24 | optische Linse |
| 6 | lichtleitende Faser | 25 | Lager |
| 7 | Ummantelung | 26 | Dichtring |
| 8 | Klebemittel | 27 | Abstandshalter |
| 9 | Ummantelung /Schrumpfschlauch | 28 | Abdeckung |
| 10 | Fassung / Hülse | | |
| 11 | Endungs-Fläche | 30 | |
| 12 | distaler, erster Endabschnitt | | |
| 13 | Proximaler, zweiter Endabschnitt | 40 | Kabel |
| 14 | lichtabgebender Endabschnitt | | |
| 15 | Hauptstrang | 50 | Biegevorrichtung |
| 16.1 | Nebenstrang | 51 | Gehäuse |
| 16.2 | Nebenstrang | 52 | Andrück- oder Biegewalze |
| 16.3 | Nebenstrang | 53 | Umlenkwalze |
| | | 54 | Querträger |
| | | 55.1 | erster Schwenkarm |
| | | 55.2 | zweiter Schwenkarm |
| M | Mittelachse | 56 | Ausnehmung |
| r | Biegeradius | 57.1 | dritter Schwenkarm |
| | | 57.2 | vierter Schwenkarm |
| | | 58, 59, 60 | Feststellschraube |
| 600 | Glaskern | 61.1 | Aufnahmebecher |
| 601 | Glasmantel | 61.2 | Aufnahmebecher |
| 602 | Trennschicht | 62 | Führungsstift |
| 700 | Kunststofffaser | | |

## Patentansprüche

1. Lichtleiter (1) mit einem Bündel (2) von lichtleitenden Fasern (6) zum Übertragen von Licht einer an einem ersten Endabschnitt (13) des Lichtleiters (1) ankoppelbaren Lichtquelle zu einem zweiten Endabschnitt (12) des Lichtleiters (1), wobei der Lichtleiter (1) einen ersten Abschnitt (3) und einen als Verbundabschnitt (5) ausgebildeten gekrümmten zweiten Abschnitt (4) aufweist,
**dadurch gekennzeichnet, dass**
die lichtleitenden Fasern (6) in dem zweiten Abschnitt (4) mittels eines ausgehärteten Klebemittels (8) bei Aufrechterhaltung der Krümmung im Wesentlichen spannungsfrei gegeneinander fixiert sind,
das Klebemittel (8) derart in den zweiten Abschnitt (4) von lichtleitenden Fasern (6) eingebracht ist, dass das Klebemittel (8) wenigstens teilweise Außenumfangsflächen der lichtleitenden Fasern (6) umgibt und miteinander verbindet, so dass das Klebemittel (8) ein Stützgerüst bildet, in das einzelne oder mehrere oder alle lichtleitenden Fasern (6) eingebettet sind, um den Verbundabschnitt (5) zu bilden.

2. Lichtleiter (1) nach Anspruch 1, **dadurch gekennzeichnet, dass** auf den zweiten Abschnitt (4) ein flexibler Schlauch (7,9) zur Stabilisierung des zweiten Abschnitts (4) aufgebracht ist.

3. Lichtleiter (1) nach einem der voranstehenden Ansprüche, **dadurch gekennzeichnet, dass** an dem ersten oder dem zweiten Endabschnitt (13, 12) des Lichtleiters (1) eine Fassung (10) zum Einsetzen in eine Aufnahme (23) zur mechanischen Befestigung des Lichtleiters (1) angebracht ist.

4. Lichtleiter (1) nach Anspruch 3, **dadurch gekennzeichnet, dass** die Fassung (10) hülsenförmig ausgebildet ist und die Endungen der lichtleitenden Fasern (6) an der Austrittseite der Fassung (10) insgesamt eine konvexe und/oder konkave und/oder ebene Fläche (11) bilden, die vorzugsweise poliert/geglättet ist.

5. Lichtleiter (1) nach Anspruch 4, **dadurch gekennzeichnet, dass** die Fassung (10) eine Hülse aus Metall, Kunststoff oder eine Kombination aus Metall oder Kunststoff ist.

6. Lichtleiter (1) nach einem der Ansprüche 2 bis 5, **dadurch gekennzeichnet, dass** der flexible Schlauch (7,9) des Verbundabschnittes (5) an die Fassung (10) anliegt.

7. Lichtleiter (1) nach einem der voranstehenden Ansprüche, **dadurch gekennzeichnet, dass** der erste und zweite Abschnitt (3, 4) derart ausgebildet sind, dass die lichtleitenden Fasern (6) in dem ersten Abschnitt (3) einen ersten Flächenquerschnitt (A) aufweisen und in dem zweiten Abschnitt (4) ein erstes und zweites Nebenbündel (16.1, 16.2) bilden, in die sich die lichtleitenden Fasern (6) des ersten Abschnittes (3) Y-förmig verzweigen und jeweils einen Flächenquerschnitt (B1, B2) aufweisen, der kleiner als der erste Flächenquerschnitt (A) ist.

8. Lichtleiter (1) nach einem der voranstehenden Ansprüche, **dadurch gekennzeichnet, dass** die lichtleitenden Fasern (6) Glasfasern und/oder Kunststofffasern sind.

9. Lichtleiter (1) nach Anspruch 8, **dadurch gekennzeichnet, dass** die Glasfasern einen Glaskern (600) mit einem Durchmesser von etwa 20 bis 500 µm, der mit einem Glasmantel (601) oder Kunststoffmantel (601) umgeben ist, aufweisen.

10. Lichtleiter (1) nach einem der voranstehenden Ansprüche, **dadurch gekennzeichnet, dass** die Krümmung des Verbundabschittes (5) einen Biegeradius (r_{xy}, r_{xz}, r_{yz}) mit konstanter oder unterschiedlicher Krümmung aufweist.

11. Lichtleiter (1) nach einem der voranstehenden Ansprüche, **dadurch gekennzeichnet, dass**, um den Verbundabschnitt (5) zu bilden, das Klebemittel (8) ein Stützgerüst bildet, in das einzelne oder mehrere oder alle lichtleitenden Fasern (6) eingebettet sind, so dass keine zusätzlichen Stützvorrichtungen, insbesondere in einem Gehäuse, für den Verbundabschnitt erforderlich sind.

12. Beleuchtungsvorrichtung mit einem Lichtleiters (1) nach einem der voranstehenden Ansprüche, **dadurch gekennzeichnet, dass** der Lichtleiter (1) in einer medizinischen Vorrichtung vorgesehen ist, insbesondere in einer Stirnlampe, einem Endoskop, einem Exoskop, einem Beleuchtungsschaft (20a) oder einem Beleuchtungsstab (20b) ohne oder mit einer Schwenkoptik (21,24).

13. Verfahren zum Biegen eines aus lichtleitenden Fasern (6) gebildeten Lichtleiters (1) mit einem ersten Abschnitt (3) und einem gekrümmten als Verbundabschnitt ausgebildeten zweiten Abschnitt (4), **gekennzeichnet durch** die Schritte:
■ Benetzen von lichtleitenden Fasern (6) des als Verbundabschnitt (5) herzustellenden zweiten Abschnitts (5) mit einem Klebemittel(8) in einem pastösen Zustand,
■ anschließend Krümmen des zweiten Abschnittes (4) in eine vorbestimmte Biegungsform, wobei das Klebemittel (8) noch in einem pastösen Zustand ist,
■ Aushärten des Klebemittels bei Aufrechterhaltung der Krümmung des zweiten Abschnittes (4), bis der zweite Abschnitt (4) den formstabilen Verbundabschnitt (5) mit vorbestimmter Biegungsform bildet.

14. Verfahren nach Anspruch 13, **dadurch gekennzeichnet, dass** vor dem Aufbringen des Klebemittels (8) ein flexibler Schlauch, insbesondere ein Schrumpfschlauch (7, 9) über einen neben dem zweiten Abschnitt gelegenen Abschnitt der lichtleitenden Fasern (6) gezogen wird und nach dem Benetzen mit dem Klebemittel (8) der lichtleitenden Faser (6) der flexible Schlauch, insbesondere Schrumpfschlauch (7,9) über den mit Klebstoff benetzten zweiten Abschnitt (4) gezogen wird.

15. Lichtleiter (1) mit einem Bündel (2) von lichtleitenden Fasern (6) mit einem gekrümmten Abschnitt (4), in dem die lichtleitenden Fasern (6) mittels eines ausgehärteten Klebemittels wenigstens teilweise spannungsfrei gegeneinander fixiert sind, hergestellt nach einem Verfahren nach einem der Ansprüche 13 bis 14.
